(19) Europäisches Patentamt
European Patent Office
Office européen des brevets

(11) **EP 4 674 949 A1**

(12) **EUROPEAN PATENT APPLICATION**
published in accordance with Art. 153(4) EPC

(43) Date of publication:
07.01.2026 Bulletin 2026/02

(21) Application number: 24853012.3

(22) Date of filing: 20.08.2024

(51) International Patent Classification (IPC):
*C12N 1/20* (2006.01)  *A61K 35/747* (2015.01)

(86) International application number:
PCT/CN2024/113374

(87) International publication number:
WO 2025/241334 (27.11.2025 Gazette 2025/48)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 23.05.2024 CN 202410643009

(71) Applicant: INNER MONGOLIA MENGNIU DAIRY
(GROUP) CO., LTD.
Hohhot, Inner Mongolia 011500 (CN)

(72) Inventors:
• XUE, Zhengsheng
  Inner Mongolia 011500 (CN)
• MAO, Yuejian
  Inner Mongolia 011500 (CN)

• ZHANG, Xuguang
  Inner Mongolia 011500 (CN)
• YANG, Jing
  Inner Mongolia 011500 (CN)
• ZHANG, Meiling
  Inner Mongolia 011500 (CN)
• YANG, Yongmei
  Inner Mongolia 011500 (CN)
• ZHU, Chenxu
  Inner Mongolia 011500 (CN)

(74) Representative: Murgitroyd & Company
165-169 Scotland Street
Glasgow G5 8PL (GB)

Remarks:
The complete document including Reference
Table(s) and the Sequence Listing(s) can be
downloaded from the EPO website

(54) **GROWTH-PROMOTING PROBIOTIC AND USE THEREOF**

(57) The invention relates to a growth-promoting probiotic and use thereof, in particular to *Lactiplantibacillus plantarum* and use thereof. The *Lactiplantibacillus plantarum* shows a good tolerance and safety, and can be used for preparing food raw materials, probiotic powder, food additives, feed additives, pharmaceuticals, liquid drinks, foods and nutraceuticals. The *Lactiplantibacillus plantarum* or a formulation containing the *Lactiplantibacillus plantarum* can promote the growth of a subject.

Fig. 4

EP 4 674 949 A1

## Description

### Technical field

The present disclosure relates to the fields of biotechnology and food, in particular to a probiotic strain and use thereof for promoting growth.

### Background of the disclosure

In recent years, the role of gut microbes in human health has been widely concerned. It has been found in studies that gut microbes play a crucial role in host immune system development, nutrient uptake and metabolic homeostasis maintenance.

Children with delayed development usually show growth retardation, developmental retardation and high morbidity etc. Some studies have shown that children who are delivered by caesarean section or fed with infant formula lack the flora inherited from their mothers, which leads to congenital low species and quantity of flora in the body, in turn leading to negative effects such as decreased diversity of intestinal flora, low immunity and growth retardation. Intestinal flora disorder is associated with growth retardation in children. Transplantation of flora from malnourished children into germ-free mice resulted in weight loss and metabolic disorders in the germ-free mice. These results demonstrate the causal relationship between microorganisms and malnutrition, and the positive effects of regulating the composition of the gut microflora on human metabolism and growth.

Probiotics are a class of active microorganisms that, when ingested in sufficient amounts, are capable of producing beneficial effects on the host. Probiotics have been used by many researchers to promote the growth and development in animals, and some probiotics such as *Lactobacillus acidophilus, Enterococcus faecium* and *Escherichia coli* strains have been independently demonstrated in different animals to promote growth, increase body weight gain, prevent pathogen colonization, and inhibit muscle atrophy. Probiotics have been found to enhance the metabolic utilization of nutrients and improve feed efficiency through the production of digestive enzymes such as amylase or B vitamins. Probiotics and their cell wall components also have been shown to promote growth in chronically malnourished mice and fruit flies by improving growth hormone sensitivity in peripheral tissues and increasing circulating levels of IGF-1. Probiotic supplementation is one of the strong candidates with the ability to promote host growth.

Childhood growth is closely linked to the health of intestinal flora, and a meta-analysis have found that some clinical trial studies have demonstrated that probiotic supplementation can increase the weight and height in normal children at the age of 0-59 months, and finding from non-clinical settings have also shown that probiotics can promote growth in malnourished children. Most current probiotic treatments focus on the growth of diseased children, while there is a paucity of studies evaluating the effects of probiotics on the growth and development of normal children.

In addition, the composition of human intestinal flora varies significantly across the globe due to geography, genetics and dietary habits etc. It has been found in studies that the diversity of intestinal flora in the Chinese population is significantly higher than that in the Western population, and there are significant differences in the composition of dominant intestinal flora between them. This makes imported probiotic strains may not be fully adapted to the intestinal environment of the Chinese people, and may have low colonization efficiency or even no beneficial effect.

Therefore, it is of great practical significance to develop a probiotic strain that can promote the growth and development of normal children and is more suitable for intestinal flora of Chinese people.

### Summary of the disclosure

As there are significant differences in the composition of intestinal flora of populations from different regions. The present disclosure aims to screen out a new growth-promoting probiotic strain which conforms with the normal diet or nutrition background of China. The present disclosure explores the growth-promoting effect of the strain in the larvae of different model organisms (including Drosophila and zebrafish), to explore its effect on growth and development of the host under the normal nutrition background, and fill the technical gaps of domestic and foreign markets.

In addition, the present disclosure compares the effect of the probiotic strain under different nutrition backgrounds (normal protein level and low protein level) to provide theoretical basis for the rational application of the probiotic strain and the development of downstream products.

The present disclosure aims to evaluate the growth-promoting effect of a probiotic strain in different animal models and to investigate its mechanism of action as follows:

1. Isolation, identification and safety evaluation of a probiotic strain
In the present disclosure, a strain of *Lactiplantibacillus plantarum* is isolated from the Chinese traditional fermented foods. The strain is identified as *Lactiplantibacillus plantarum* using phenotypic identification, molecular biological identification and phylogenetic analysis. Probiotics need to pass through and colonize in the gastrointestinal tract before they can perform their beneficial functions. In this experiment, the acid and bile salt tolerance of the strain are tested to evaluate the ability of the strain to pass through the gastrointestinal tract and hemolysis and drug sensitivity tests are performed to assess the safety of the probiotic strain.

2. Growth-promoting effects of the probiotic strain in different animal models

According to the present disclosure, the effect of the probiotic strain on the growth performance of different model animals is explored under normal nutrition background, by detecting the larval size, pupation time and eclosion time in Drosophila, as well as weight, length and the rate of weight gain in zebrafish.

3. Effects of the probiotic strain on the host growth under different nutritional backgrounds

The effects of probiotics may vary under different nutritional backgrounds. The present disclosure sets two different nutritional backgrounds, normal protein level and low protein level, to compare the effects of the probiotic strain under different nutritional backgrounds.

In a first aspect, the present disclosure provides *Lactiplantibacillus plantarum* Hi188, which is deposited with the Accession number CGMCC No.30250.

The present disclosure also provides a formulation comprising *Lactiplantibacillus plantarum* Hi188.

The formulation comprising *Lactiplantibacillus plantarum* Hil88 is one or more of a food raw material, a probiotic powder, a food additive, a feed additive, a pharmaceutical, a liquid drink, a food or a nutraceutical.

The formulation comprising *Lactiplantibacillus plantarum* Hil88 is a solid or a liquid formulation. The formulation comprises *Lactiplantibacillus plantarum* Hi188 in an amount of $1.0 \times 10^5$ CFU - $1.0 \times 10^{12}$ CFU/mL when it is a liquid formulation. The formulation comprises *Lactiplantibacillus plantarum* Hi188 in an amount of $1.0 \times 10^5$ CFU -$1.0 \times 10^{12}$ CFU /g when it is a solid formulation.

The formulation comprising *Lactiplantibacillus plantarum* Hil88, which further comprises an excipient, and said excipient comprises one or more of inulin, fructo-oligosaccharide, skim milk powder, desalted whey powder, lactoferrin, casein phosphopeptide or hydrolyzed egg yolk powder.

In another aspect, the present disclosure provides the use of *Lactiplantibacillus plantarum* Hi188 for the preparation of a formulation or kit for promoting the growth of a subject.

In an embodiment, the amount of said *Lactiplantibacillus plantarum* Hi188 in the formulation or the kit for promoting growth in a subject is in range of $1.0 \times 10^5$ CFU -$1.0 \times 10^{12}$ CFU/mL or $1.0 \times 10^5$ CFU -$1.0 \times 10^{12}$ CFU/g.

In a particular embodiment of the present disclosure, said *Lactiplantibacillus plantarum* Hi188 or formulation comprising *Lactiplantibacillus plantarum* Hi188 may be present as an active ingredient in a kit. The kit refers to a product in any form (e.g., a nutraceutical kit, a pharmaceutical kit, etc.) comprising the active ingredient, which optionally also comprising a containment device, an outer wrapper, and/or an instruction for use. The instruction for use may be used to provide information on the administration of the active ingredient, such as the amount to be administered, the frequency of administration, or to indicate the use of the active ingredient, such as for promoting an increase in the length or height of a subject, for promoting an increase in the weight of a subject, or for promoting the development of a subject.

The present disclosure also provides the use of a formulation comprising *Lactiplantibacillus plantarum* Hi188 for promoting growth in a subject.

In an embodiment, promoting growth of the subject comprises one or more of promoting an increase in body length of the subject, promoting an increase in body weight of the subject, and promoting development of the subject.

In an embodiment, the subject is a Drosophila, a zebrafish, or a mammal.

In an embodiment, the mammal is a mouse or a human.

In the present disclosure, the normal protein refers to the level of protein routinely ingested in the prior art for maintaining the normal growth and development of an organism. The low protein level refers to a level of protein that significantly interferes with the normal growth and development of an organism, such as a level of protein that is less than 20%-60% of the normal protein.

The development and application of probiotics in the era of functional foods are the current research focus, which meets the demand for functional and healthy food. Among them, functional products based on probiotics are welcomed by the masses. The beneficial effects of the present disclosure are that it not only focuses on the growth-promoting effect of probiotics on larve organisms under normal nutritional backgrounds, but also focuses on the effects of probiotics under different nutritional backgrounds, which enlarges the scope of rational application of probiotics, and provides an important reference value to the research of growth-promoting probiotics for human beings. Furthermore, it also provides a solution to improve the height problem of children with growth retardation, and also provides more options for normal children with higher height expectations.

**Brief description of the drawings**

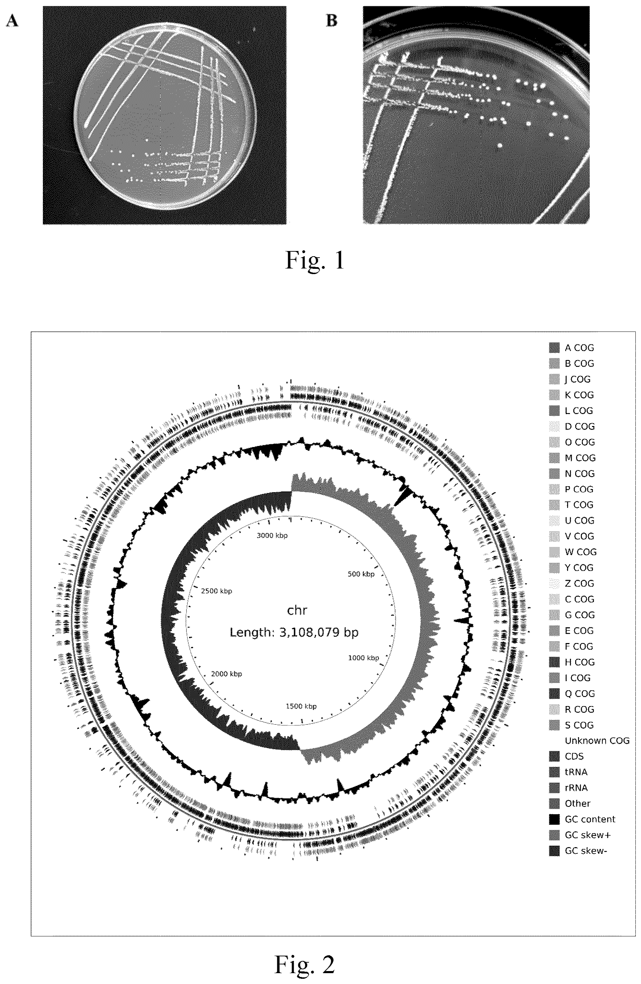

Fig. 1 shows the colony morphology of *Lactiplantibacillus plantarum* Hi188, where Panel A is an overall top view; Panel B is a partial view.

Fig. 2 shows the genomic circle of *Lactiplantibacillus plantarum* Hi188.

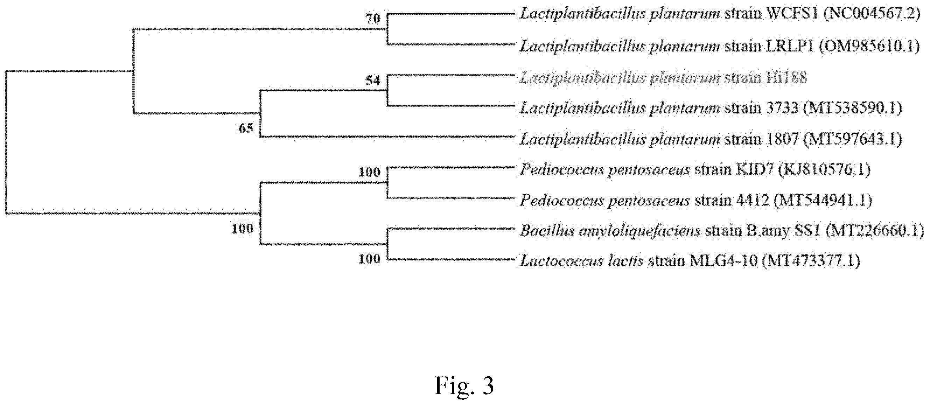

Fig. 3 shows the phylogenetic tree of *Lactiplantibacillus plantarum* Hi188.

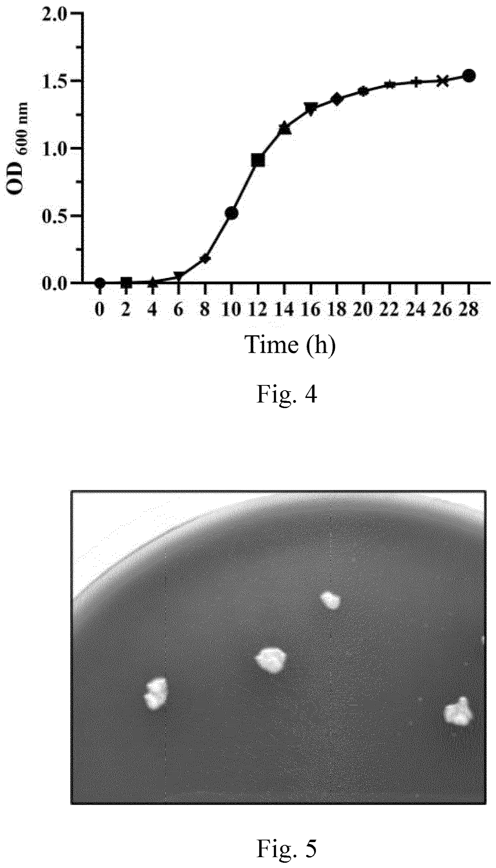

Fig. 4 shows the growth curve of *Lactiplantibacillus plantarum* Hi188.

Fig. 5 shows the hemolytic assay of *Lactiplantibacillus plantarum* Hi188.

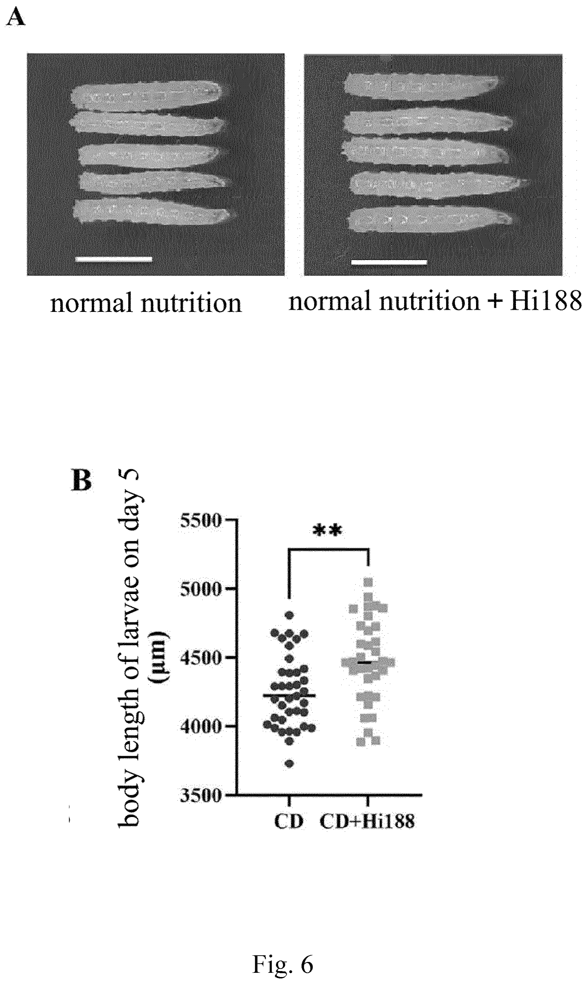

Fig. 6 shows the effect of *Lactiplantibacillus plantarum* Hi188 on the growth of Drosophila larvae under normal nutrition condition. Panel A is a comparative photo of Drosophila larvae from groups with and without Hi188 under normal nutrition at day 5. Panel B shows the statistical comparison of body length of Drosophila from groups with and without Hi188 under normal nutrition at day 5. "CD" represents the normal nutrition group, "CD+Hi188" represents the group fed with normal nutrition and Hi188 at the dosage of $10^9$ CFU. ** indicates P < 0.01.

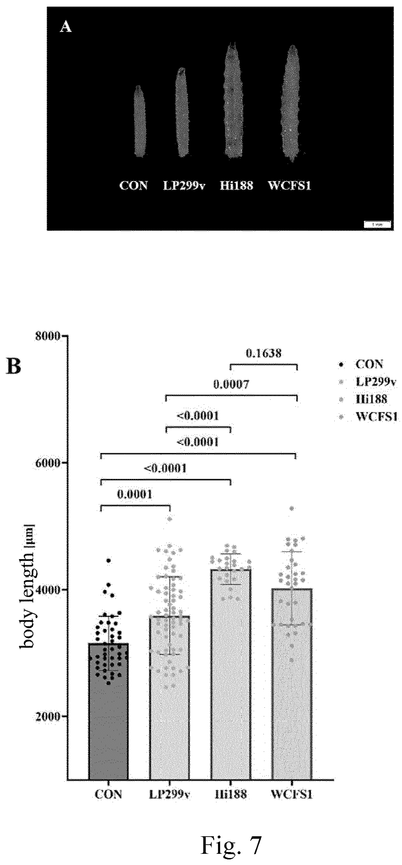

Fig. 7 shows a comparison of the growth effect of *Lactiplantibacillus plantarum* Hi188, comparison strains *Lactiplantibacillus plantarum* LP299v *and Lactiplantibacillus plantarum* WCFS1 in Drosophila larvae under normal nutrition. Panel A is the comparative photograph of Drosophila larvae in CON, LP299v, Hi188, or WCFS1 group at day 5. Panel B shows the statistical comparison of the body length of Drosophila larvae at day 5 in CON, LP299v, Hi188 or WCFS1 group. CON, LP299v, Hi188, and WCFS1 represent groups with no treated, LP299v, Hi188 and WCFS1 under normal nutrition, respectively, and the dosage of LP299v, Hi188 and WCFS1 is $10^9$ CFU.

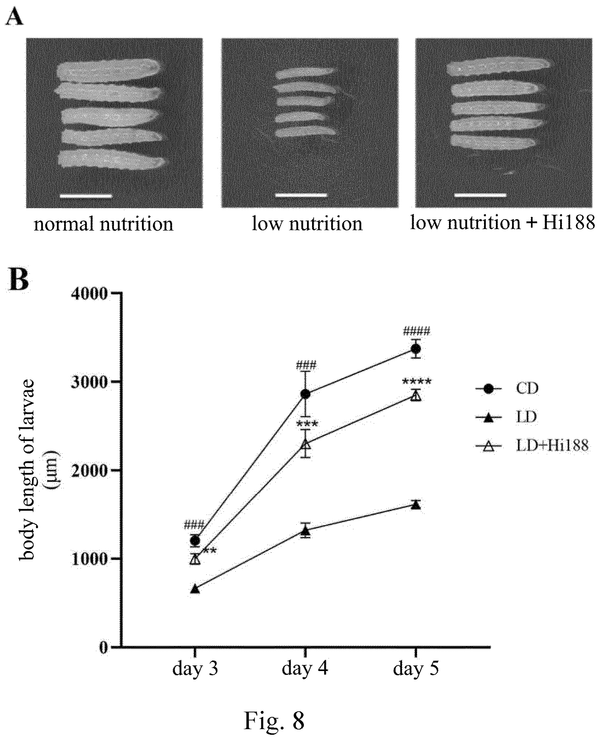

Fig. 8 shows the effect of *Lactiplantibacillus plantarum* Hi188 on the growth of Drosophila larvae under the low nutrition condition. Panel A shows the comparison photographs of Drosophila larvae with normal nutrition, low nutrition and low nutrition and Hi188 at day 5. Panel B shows the statistical comparison of body length of Drosophila larvae with normal nutrition, low nutrition and low nutrition and Hi188 from day 3 to day 5. "CD", "LD", and "LD+Hi188" represent group with normal nutrition, low nutrition and low nutrition with Hi188, respectively, and the dosage of Hi188 is $10^9$ CFU.

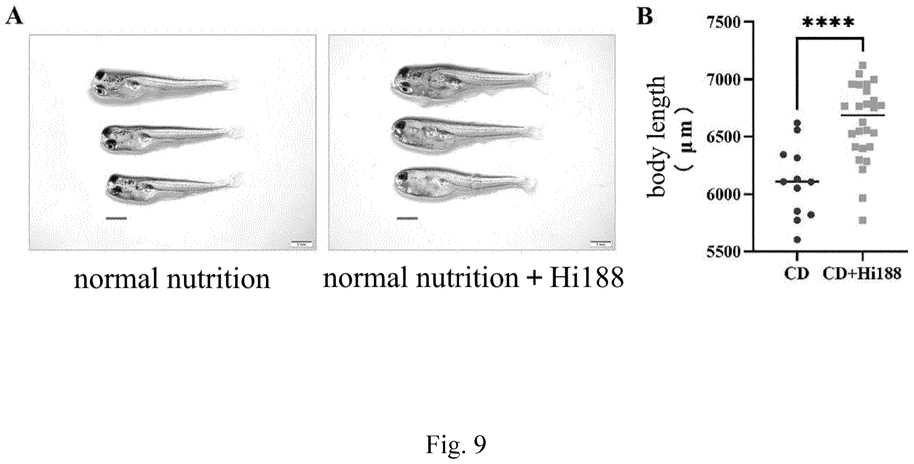

Fig. 9 shows the effect of *Lactiplantibacillus plantarum* Hi188 on the growth of zebrafishes larvae under the normal nutrition. Panel A shows the comparison photographs of zebrafishes larvae from groups with and without Hi188 under normal nutrition at week 4. Panel B shows the statistical comparison of the body length of zebrafishes larvae from groups with and without Hi188 under normal nutrition at week 4. "CD" and "CD+Hi188" represent group fed with normal nutrition and Hi188 at a dosage of $10^9$ CFU. **** indicates P < 0.0001.

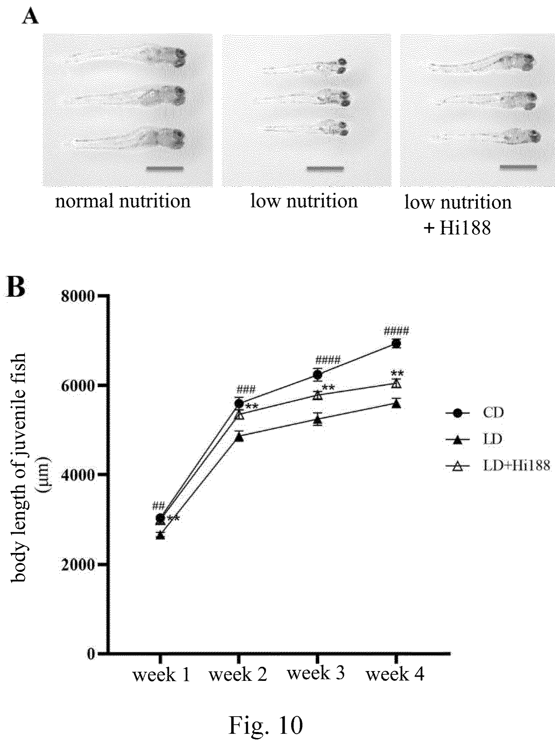

Fig. 10 shows the effect of *Lactiplantibacillus plantarum* Hi188 on the growth of zebrafishes larvae under the low nutrition. Panel A shows the comparison photographs of zebrafishes larvae with normal nutrition, low nutrition and low nutrition with Hi188 at week 4. Panel B shows the statistical comparison of the body length of zebrafishes larvae with normal nutrition, low nutrition, low nutrition with Hi188 from week 1 to week 4. "CD", "LD" and "LD+Hil88" represent group fed with normal nutrition, low nutrition, and low nutrition with Hi188, respectively. The dosage of Hi188 is $10^9$ CFU.

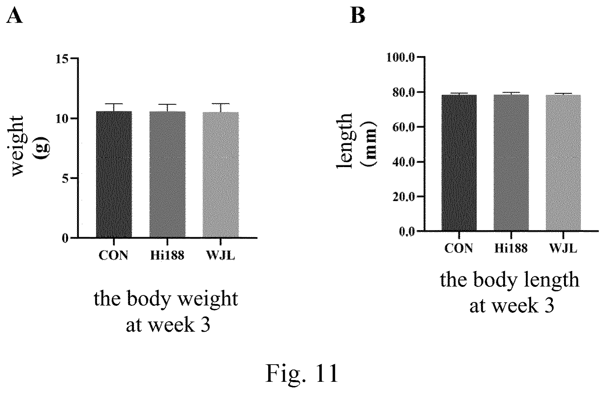

Fig. 11 shows the comparison of the body weight and length of mice at week 3. Panel A shows the comparison of the body weight of three-week-old mice in the normal control (CON), Hi188 and WJL group. Panel B shows the comparison of the body length of three-week old mice in the normal control (CON), Hi188 and WJL group.

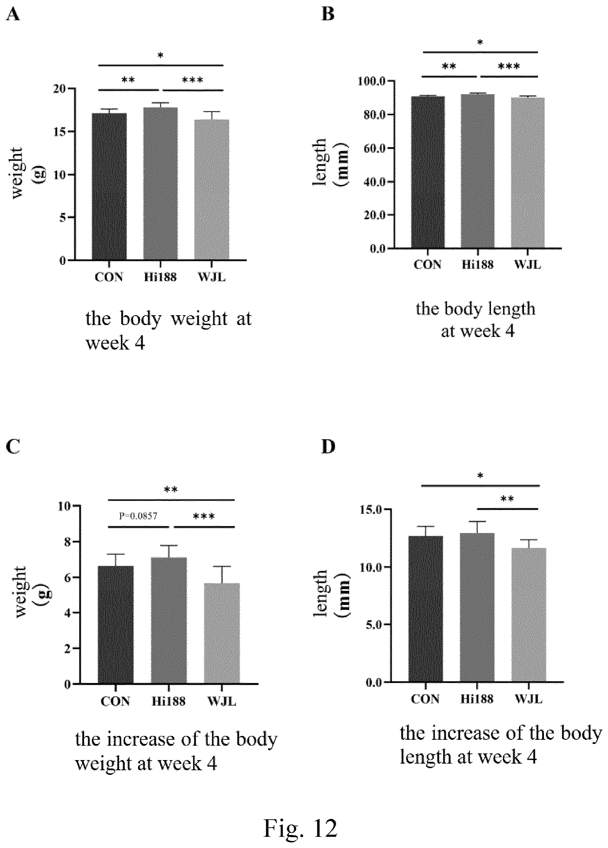

Fig. 12 shows the comparison of the body weight and length of mice at week 4. Panel A shows the comparison of the body weight of four-week-old mice in the normal control (CON), Hi188 and WJL group. Panel B shows the comparison of the body length of four-week-old mice in the normal control (CON), Hi188 and WJL group. Panel C shows the 1-week body weight gain of mice in the normal control (CON), Hi188 and WJL group. Panel D shows the 1-week body length gain of mice in the normal control (CON), Hi188 and WJL group. * indicates P < 0.05, ** indicates P < 0.01, and *** indicates P < 0.001.

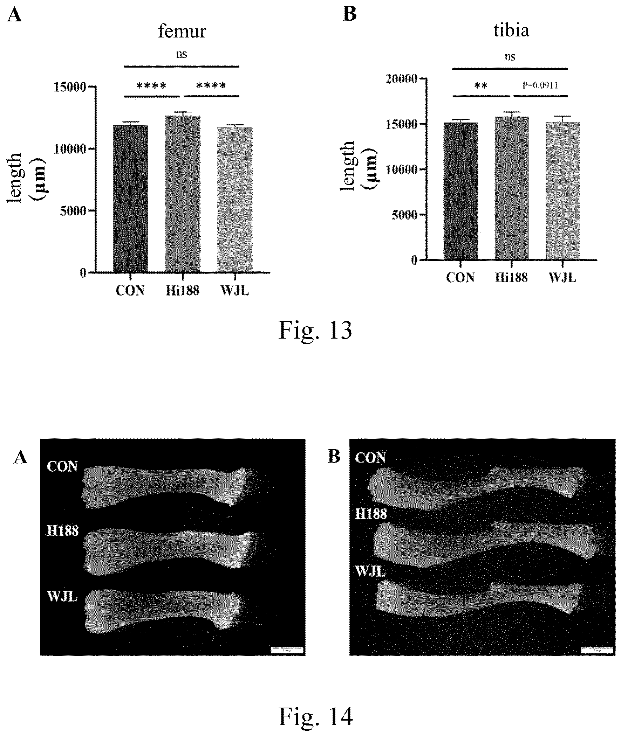

Fig. 13 shows the comparison of the femur length and the tibia length of mice at week 4. Panel A shows the comparison of the femur length of mice at week 4 in the normal control group (CON), Hi188 group and WJL group. Panel B shows the comparison of the tibia length of mice at week 4 in the normal control group (CON), Hi188 group and WJL group. ** indicates P<0.01, *** indicates P<0.001, and **** indicates P<0.0001.

Fig. 14 shows the photograph of the femurs and the tibias of mice at week 4. Panel A shows the comparison of the femurs of mice at week 4 in the normal control group (CON), Hi188 group, and WJL group. Panel B shows the comparison of the tibias of mice at week 4 in the normal control group (CON), the Hi188 group, and the WJL group.

**Deposit description**

Taxonomy: *Lactiplantibacillus plantarum*
Name: Hi188
Latin name: *Lactiplantibacillus plantarum*
Depository: China General Microbiological Culture Collection Center
Abbreviation of depository: CGMCC
Address: No. 3, Yard 1, West Beichen Road, Chaoyang District, Beijing, China.
Deposition date: April 2, 2024
Accession Number of the depository: CGMCC No. 30250.

**Detailed description of the disclosure**

In order to better understand the object, the technical solution and the advantages of the present disclosure, the present disclosure will be further described in detail below with reference to specific examples and the accompanying drawings.

The methods used in the following examples are conventional methods if not otherwise specified, and the reagents used are commercially available if not otherwise specified.

In Examples of the present disclosure, the said Drosophila and zebrafish are conventional model organisms of the prior art, which are commercially available. For example, the wild-type Canton Special strain of *Drosophila melanogaster* was purchased from the National Drosophila Resource Center (Shanghai, China), and the zebrafish AB line was purchased from the China Zebrafish Resource Center (Wuhan, China).

**Example 1. Isolation and Identification of *Lactiplantibacillus plantarum* Hi188**

1) Isolation and purification

2.5 mL of the fluid of Chinese cheese (Wujing Town, Minhang District, Shanghai, China) was added into a 10 mL test tube, and then 2.5 mL of 1 × PBS buffer was added and centrifuged at 3000 r/min for 5 min. 100 µL of the supernatant was added into 40 mL of MRS broth medium (purchased from Hangzhou BaiSi Biotechnology Co., Ltd.), and incubated overnight at a 30 °C with shaking at 200 r/min. The incubated bacterial liquid was gradient diluted in 10- fold with the sterile 1 × PBS buffer. 100 µL of the $10^4$-fold diluted solution was spread on MRS solid medium (1.5% agar was added into the MRS broth medium) with a spreader, and then incubated in inverted position at 30 °C to obtain single colonies of lactic acid bacteria. Single colonies with different morphology were selected from the plate, and after repeated delinenation for isolation and purification, they were placed in a constant temperature incubator at 30 °C for 48h of continuous culture. The above steps were repeated until pure colonies were obtained.

2) Passage and cryopreservation

The colonies on the MRS solid medium were scraped using an inoculating loop, inoculated into 10 mL of the MRS broth medium, and incubated in a shaker at 30°C and 200 r/min for 12 h. Then, the sterilized 60% glycerol was mixed with the broth in a volume ratio of 1:3 to make the final concentration of glycerol 15%, and then blown and mixed well, and the strains were preserved at -80 °C.

3) Identification and phylogenetic analysis of the strain

A small number of frozen bacteria was dipped into MRS solid medium for plate delinenation by three-zone delineation to observe the single colony morphology. The morphologies of single colonies were observed. On the normal MRS solid medium, *Lactiplantibacillus plantarum* Hi188 appeared as a creamy white, round, uniformly-sized, slightly convex colony with a moist and smooth surface and neat edges (Fig. 1).

Fresh single-strain bacterial solution was used as the template in a PCR amplification system for PCR amplification. The primer sequences of the bacterial 16s rRNA gene of the bacterium used were as follows: forward primer 27F: 5'-AGAGTTTGATCMTGGCTCAG-3' (SEQ ID NO: 1); reverse primer 1492R: 5'-GGTTACCTTGTTACGA CTT-3' (SEQ ID NO: 2). A homologous sequence alignment on the sequencing result was performed by using BLAST software in the NCBI database. A phylogenetic tree was established by MEGA software according to the alignment result. The isolate was named *Lactiplantibacillus plantarum* Hil88, and the sequence of 16s rRNA gene was obtained by 16S rRNA gene sequencing as follows:

GCAGTCGAACGAACTCTGGTATTGATTGGTGCTTGCATCATGATTTA

CATTTGAGTGAGTGGCGAACTGGTGAGTAACACGTGGGAAACCTGCCCAG

AAGCGGGGGATAACACCTGGAAACAGATGCTAATACCGCATAACAACTTG

GACCGCATGGTCCGAGCTTGAAAGATGGCTTCGGCTATCACTTTTGGATGG

TCCCGCGGCGTATTAGCTAGATGGTGGGGTAACGGCTCACCATGGCAATGA

TACGTAGCCGACCTGAGAGGGTAATCGGCCACATTGGGACTGAGACACGG

CCCAAACTCCTACGGGAGGCAGCAGTAGGGAATCTTCCACAATGGACGAA

AGTCTGATGGAGCAACGCCGCGTGAGTGAAGAAGGGTTTCGGCTCGTAAA

ACTCTGTTGTTAAAGAAGAACATATCTGAGAGTAACTGTTCAGGTATTGAC

GGTATTTAACCAGAAAGCCACGGCTAACTACGTGCCAGCAGCCGCGGTAAT

ACGTAGGTGGCAAGCGTTGTCCGGATTTATTGGGCGTAAAGCGAGCGCAG

GCGGTTTTTAAGTCTGATGTGAAAGCCTTCGGCTCAACCGAAGAAGTGCA

TCGGAAACTGGGAAACTTGAGTGCAGAAGAGGACAGTGGAACTCCATGT

GTAGCGGTGAAATGCGTAGATATATGGAAGAACACCAGTGGCGAAGGCGG

CTGTCTGGTCTGTAACTGACGCTGAGGCTCGAAAGTATGGGTAGCAAACA

GGATTAGATACCCTGGTAGTCCATACCGTAAACGATGAATGCTAAGTGTTG

GAGGGTTTCCGCCCTTCAGTGCTGCAGCTAACGCATTAAGCATTCCGCCTG

GGGAGTACGGCCGCAAGGCTGAAACTCAAAGGAATTGACGGGGGCCCGC

ACAAGCGGTGGAGCATGTGGTTTAATTCGAAGCTACGCGAAGAACCTTAC

CAGGTCTTGACATACTATGCAAATCTAAGAGATTAGACGTTCCCTTCGGGG

ACATGGATACAGGTGGTGCATGGTTGTCGTCAGCTCGTGTCGTGAGATGTT

GGGTTAAGTCCCGCAACGAGCGCAACCCTTATTATCAGTTGCCAGCATTAA

GTTGGGCACTCTGGTGAGACTGCCGGTGACAAACCGGAGGAAGGTGGGG

ATGACGTCAAATCATCATGCCCCTTATGACCTGGGCTACACACGTGCTACA

ATGGATGGTACAACGAGTTGCGAACTCGCGAGAGTAAGCTAATCTCTTAAA

GCCATTCTCAGTTCGGATTGTAGGCTGCAACTCGCCTACATGAAGTCGGAA

TCGCTAGTAATCGCGGATCAGCATGCCGCGGTGAATACGTTCCCGGGCCTT

GTACACACCGCCCGTCACACCATGAGAGTTTGTAACACCCAAAGTCGGTG

GGGTAACCTTTTAGGAACCAGCCGCCTAA (SEQ ID NO: 3).

The results of the whole-genome sequencing and analysis of the strain show that Hi188 consists of one chromosomal DNA and 10 plasmid DNAs, of which the chromosomal sequence length is 3,108,079 bp and the GC content is 44.75 (Fig. 2). The phylogenetic tree of Hi188 is shown in Fig. 3, where the Hi188 strain is clustered with four other strains of *Lactiplantibacillus plantarum.* Hi188 is identified as *Lactiplantibacillus plantarum,* which belongs to the strains permitted for use in food on the "List of Strains that Can Be Used in Food".

**Example 2. Properties of *Lactiplantibacillus plantarum* Hi188**

1) Determination of growth curve

The growth curve of the strain was observed during the continuous incubation at 37 °C for 24h in the MRS broth medium. A single colony was inoculated into a centrifuge tube with 40 mL of the MRS broth medium and incubated in a shaker at 37 °C and 200 r/min. Every 2 h, 200 $\mu$L of the well-mixed bacterial suspension was taken out, and blank medium was used as the control, the absorbance value $OD_{600nm}$ was measured at a wavelength of 600 nm in triplicate, and then the growth curve was drawn. The logarithmic growth phase of the strain Hi188 was reached at 6h and the stable phase was reached at about 22h (Fig. 4).

2) Determination of tolerance to bile salts

The bile salt concentration of intestinal fluid was 0.03% -0.3%, for which the bile salt resistant of Hi1188 was tested under the condition of 0.3% bile salts.

MRS broth medium containing 0.3% bile salts was first prepared with MRS broth and bovine bile salts (purchased from Beijing Solarbio Science & Technology Co., Ltd.), and then sterilized in an autoclave at 121 °C for 15 min and cooled down for future use. The strain was cultured in the MRS broth for 8h, and then inoculated into 40 mL of prepared MRS broth medium or MRS broth medium containing 0.3% bile salts at an inoculum rate of 1%, respectively. The MRS broth medium containing 0.3% bile salts and bacteria was used as the treatment group; the MRS broth medium containing 0.3% bile salts (without the bacteria) was used as the negative background group for the treatment group; the MRS broth medium (without the bile salts) inoculated with the bacteria was used as the positive control; and the MRS broth medium without the bacteria (without the bile salts) was used as the negative background group for the positive control. Each treatment and control were in triplicate, and cultured in a shaker at 37 °C and 200 r/min for 3 h. The OD values of the bacterial solution were detected at 0 h, 1 h, 2 h and 3 h, respectively. The survival rate was calculated using the following formula:

Survival rate (%) = (the OD value of the treatment group - the OD value of the negative background of the treatment group) / (the OD value of the positive control - the OD value of the negative background of the positive control group) $\times$ 100%

The survival rates of the strain at 1 h, 2 h and 3 h are shown in Table 1 below. The results show that *Lactiplantibacillus plantarum* Hi188 has good tolerance to bile salts.

Table 1. Survival rate of *Lactiplantibacillus plantarum* Hil88 tolerant in presence of bile salts

|  | time | | |
|---|---|---|---|
| tolerance condition | 1 h | 2 h | 3 h |
| 0.3% bile salts | 56.75% | 48.42% | 64.22% |

3) Evaluation of tolerance to the artificial gastric fluid and intestinal fluid

The probiotics toned to pass through the gastrointestinal tract and colonized before they can perform their beneficial functions. In this study, we tested the tolerance of Hi188 in artificial gastric fluid at pH 3.0 (purchased from Shanghai Canspec Scientific Instruments Co., Ltd.) and in artificial small intestinal fluid at pH 6.8 (purchased from Shanghai Chuangsai Technology Co., Ltd.). After culturing Hi188 for 24 h, 1 mL of the bacterial solution was inoculated into 9 ml of artificial gastric fluid or small intestinal fluid. The artificial gastric fluid or the small intestinal fluid incubated with the bacteria was used as the treatment group. The blank artificial gastric fluid or small intestinal fluid without the bacteria was used as the negative control. Each treatment and control were incubated in triplicate at 37 °C and 200 r/min for 3 h. The number of viable bacteria was counted at 0 h, 1 h, 2 h and 3 h, respectively. The survival rate was calculated using the following formula:

Survival rate (%) = the number of viable bacteria of the treatment group / the number of viable bacteria of the treatment group at 0 h $\times$ 100%

Table 2. Survival rate of *Lactiplantibacillus plantarum* Hil88 tolerant in presence of the artificial gastrointestinal fluids

|  | survival rate | | | |
|---|---|---|---|---|
| time | 0 h | 1 h | 2 h | 3 h |
| artificial gastric fluid | 100.00% | 69.97% | 57.76% | 56.93% |
| artificial intestinal fluid | 100.00% | 99.82% | 85.28% | 77.13% |

The results are shown in Table 2. After 3h treatments, the survival rate of *Lactiplantibacillus plantarum* Hi188 in artificial gastric fluid and artificial small intestinal fluid can reach more than 50%, which indicates that *Lactiplantibacillus plantarum* Hi188 has a strong survival ability in the artificial gastrointestinal fluids.

**Example 3. Safety Evaluation of *Lactiplantibacillus plantarum* Hi188**

1) Hemolysis assay

Single-colony of the isolated Hi188 strain were inoculated on the blood plates (purchased from Anhui Wuhu Oak Biotechnology Co., Ltd). The strain was cultured in an incubator at 37 °C for 36 h to observe whether hemolysis occurred. As shown in Fig. 5, *Lactiplantibacillus plantarum* Hi188 was a white smooth colony without hemolysis on the blood plate, which indicated that Hi188 had no obvious pathogenicity and higher safety.

2) Determination of the MIC value

The microdilution method was used to study the antibiotic resistance of the strains to Ampicillin (Amp), Gentamicin (Gen), Kanamycin (Kan), Erythromycin (Ery), Clindamycin (Cli), Tetracycline (Tet) and Chloramphenicol (Chl). The determination was performed according to the standard method in ISO 10932/IDF223. The minimum inhibitory concentration (MIC) was used to represent the antibiotic resistance.

The detection range of mass concentration was 0.5~ 256 μg/mL. The antibiotic stock solution was prepared using various antibiotics, among which the ampicillin sodium, kanamycin sulfate and chloramphenicol were purchased from Easen Biotechnology (Shanghai) Co., Ltd.; gentamicin and tetracycline hydrochloride were purchased from Shanghai YuanYe Bio-Technology Co., Ltd.; erythromycin was purchased from Shanghai Macklin Biochemical Technology Co., Ltd.; and clindamycin was purchased from Shanghai Aladdin Biochemical Technology Co., Ltd. The antibiotics were weighted accurately according to the titers of reagents used, dissolved in an appropriate solvent, and mixed by shaking.

LSM liquid medium was composed of 90% IST liquid medium (purchased from Shandong Tuopu Biol-engineering Co., Ltd.) and 10% MRS broth (purchased from Hangzhou Baisi Biotechnology Co., Ltd.). The medium was used for the determination of MIC values. The prepared high-concentration antibiotic stock solutions were diluted to twice the maximum concentration using the LSM liquid medium for the assay.

The $OD_{600nm}$ of the activated strain was measured. The broth was diluted with LSM medium in a corresponding fold, and then the 1% inoculum was added into the diluted antibiotics solution with different concentration gradients, so that the number of inoculated viable bacteria was $3 \times 10^5$ CFU /mL. After incubated 24 h in 96-well microtiter plates at 37 °C the $OD_{600nm}$ values were measured using a microtiter plate spectrophotometer. The minimum concentration of the antibiotics corresponding to non-growth of the bacteria was considered as the MIC value of the strain. *Lactiplantibacillus plantarum* LP[WCFS1] (purchased from China General Microbiological Culture Collection Center) was used as the control strain when the MIC value was determined. The broth with bacteria was used as a positive control, and the broth without bacteria was used as a negative control. Hi188 and controls were set up in triplicate. When the results of parallel groups were inconsistent, the experiment was repeated.

The results of the MIC assay are shown in Table 3, where "-" indicates that the concentration of the antibiotic is bacteriostatic, and "+" indicates that the concentration of the antibiotic is not bacteriostatic. The results of the resistance of Hi188 and LP[WCFS1] to various type of antibiotics are similar. Hi188 is more sensitive to kanamycin, tetracycline and chloramphenicol than that of LP[WCFS1].

Table 3. Comparison of the antibiotic resistances between *Lactiplantibacillus plantarum* Hi188 and LP[WCFS1]

| antibiotic | group | antibiotic concentration (μg/ml) | | | | | | | | | | positive control | negative control | MIC value | critical value | resistant |
|---|---|---|---|---|---|---|---|---|---|---|---|---|---|---|---|---|
| | | 256 | 128 | 64 | 32 | 16 | 8 | 4 | 2 | 1 | 0.5 | | | | | |
| Ampicillin | Hi188 | - | - | - | - | - | - | - | + | + | + | + | - | 4 | 2 | R |
| | LP WCFS1 | - | - | - | - | - | - | - | + | + | + | + | - | 4 | | R |
| Gentamicin | Hi188 | - | - | - | - | - | - | - | - | - | - | + | - | <0.5 | 16 | S |
| | LP WCFS1 | - | - | - | - | - | - | - | - | - | - | + | - | <0.5 | | S |
| Kanamycin | Hi188 | - | - | - | - | - | - | + | + | + | + | + | - | 8 | 64 | S |
| | LP WCFS1 | - | - | - | - | - | + | + | + | + | + | + | - | 16 | | S |
| Erythromycin | Hi188 | - | - | - | - | - | - | - | - | - | - | + | - | <0.5 | 1 | S |
| | LP WCFS1 | - | - | - | - | - | - | - | - | - | - | + | - | <0.5 | | S |
| Clindamycin | Hi188 | - | - | - | - | - | - | - | - | - | - | + | - | <0.5 | 2 | S |
| | LP WCFS1 | - | - | - | - | - | - | - | - | - | - | + | - | <0.5 | | S |
| Tetracycline | Hi188 | - | - | - | - | - | - | + | + | + | + | + | - | 8 | 32 | S |
| | LP WCFS1 | - | - | - | - | - | + | + | + | + | + | + | - | 16 | | S |
| Chloramphenicol | Hi188 | - | - | - | - | - | - | - | + | + | + | + | - | 4 | 8 | S |
| | LP WCFS1 | - | - | - | - | - | - | - | - | + | + | + | - | 2 | | S |

Note: EFSA standard was used for the cut-off value. An MIC value greater than the critical value represented resistance type (R), and an MIC value less than or equal to the critical value represented sensitiveness type (S).

**Example 4. Growth-Promoting Effect of *Lactiplantibacillus plantarum* Hi188 on Drosophila**

Eggs laid by mating adult Drosophila were transferred to a sterile normal protein medium within 8 h from oviposition, and 5 parallel culture tubes were set up for each treatment. The culture medium was a yeast-corn flour culture medium. The medium was boiled for 5 min and sterilized at 121 °C for 15 min. $10^9$ CFU of *Lactiplantibacillus plantarum* Hi188 was resuspended in 200 μL of sterile water and inoculated uniformly on the medium, while the control group was added with an equal amount of sterile water, and then the eggs were placed in an incubator at 25 °C, with 12/12 hours of dark/light cycle and at a humidity of about 40%. On days 3~5, larvae in the control and Hi188 group were randomly collected daily. The larvae were photographed and the body lengths were measured by a stereo microscope using Image J software.

Two different nutritional backgrounds, normal protein level and low protein level were set up to compare the effects of probiotics under different nutritional backgrounds. In the experiment with the normal protein background, the yeast-corn flour medium was formulated as 1 L of feed containing 20 g of white sugar, 20 g of brown sugar, 80 g of corn flour, 40 g of yeast, 10 g of agar powder, and 1.5-2 % of propionic acid, in which the content of the yeast was 40 g/L. In the experiment with the low protein background, the content of the yeast in the medium was adjusted to be 10 g/L, and the other components were identical to those of the normal protein medium.

The results show that the body length of Drosophila larvae in the Hi188 group is significantly longer than that of the control group on a normal nutrition background, suggesting that *Lactiplantibacillus plantarum* Hi188 can promote the growth of Drosophila larvae (Fig. 6). *Lactiplantibacillus plantarum* LP299v (isolated from healthy human intestinal mucosa, purchased from Probi, a Swedish probiotic production company) and WCFS1 (isolated from human oral cavity saliva, purchased from China General Microbiological Culture Collection Center) were used as comparative strains. According to the comparison of the growth effect, the growth-promoting effect of *Lactiplantibacillus plantarum* Hi188 on the body length of Drosophila larvae is significantly superior to that of the control strain *Lactiplantibacillus plantarum* LP299v, and the growth-promoting effect of Hil88 is also significantly superior to that of another control strain WCFS1 (Fig. 7).

Under the background of the low protein nutrition, the photographs taken on day 5 show that the body length of Drosophila larvae in the low nutrition group is significantly lower than that of the normal nutrition group, and growth is shown to be stunted (Fig. 8A). Compared to the low-nutrition group, Drosophilia in the Hi188 group has an earlier pupation time and an earlier eclosion time. That is, the Hi188 addition alleviates the adverse effects caused by the developmental retardation in the low nutrition. In combination of the statistical results of body lengths on day 3 and day 5, $10^9$ CFU of Hi188

added to the low-nutrition feed can significantly promote the growth and development of Drosophila larvae, and alleviates the adverse effects caused by the low nutrition (Fig. 8).

Further, this Example also verified that a similar effect was observed when *Lactiplantibacillus plantarum* Hi188 was added at $1.0 \times 10^5$ CFU and $1.0 \times 10^{12}$ CFU.

**Example 5 Growth-Promoting Effect of *Lactiplantibacillus plantarum* Hi188 on Zebrafish (Danio rerio)**

The parental zebrafishes were allowed to mate for 4~6 h, and then the fish eggs were collected in saline water (1.5 mL/L of sea salt) containing 9 μL/L methylene blue. The fish eggs were grouped and cultured in saline water at 27 ± 1 °C with a dark/light cycle of 14 h/10 h in an incubator. The control group and the treatment group were set up in triplicate. Feed the fishes with yolks twice a day in the first two days after the eggs hatch Subsequently, the fishes were fed with the feed twice daily, while the Hi188 bacterial solution resuspended in sterile water was added to the culture water at the time of feeding, resulting in a final concentration of Hi188 in the culture water was $10^8$ CFU/mL in the Hi188 group. And the control group was fed with an equal amount of sterile water. From week 1 to week 4, juvenile zebrafish were photographed and body length were measured weekly by a stereomicroscope using Image J software.

Two different nutritional backgrounds, normal protein level and low protein level were set up to compare the effects of probiotics under different nutritional backgrounds. In the experiment of normal protein background, the feed was formulated as 1 L of feed contained 500 g of fish meal, 300 g of starch, 20 g of fish oil, 15 g of multivitamin, 15 g of multi-mineral, 4 g of sodium carboxymethyl cellulose, 144.38 g of cellulose, 0.5 g of choline chloride, 0.02 g of dibutyl hydroxy toluene, 0.1 g of dimethyl-β-propiothetin, 1 g of calcium dihydrogen phosphate, and the protein content of the feed was determined to be 36 % by Kjeldahl method. In the experiment of low protein background, the fish meal was adjusted to 300 g, fish oil to 40 g, cellulose to 324.38 g in 1 L of the feed, and other components were the same as normal protein feed, and the protein content was determined to be 21% by Kjeldahl method.

After 4 weeks, comparing the normal nutrition group and the normal nutrition with Hi188 group, the results show that the growth and development of zebrafish larvae are obvious in the normal nutrition with Hi188 group, with widening of the eye diameter, growth of the body length, and increase of the body area, which proves that *Lactiplantibacillus plantarum* Hi188 has a growth-promoting effect on juvenile zebrafish under normal nutrition (Fig. 9).

Compared with the juvenile zebrafish in normal nutrition group, the juvenile zebrafish in low protein nutrition had a significantly smaller body length, and delayed development. Compared with the low nutrition group, the juvenile zebrafish in group fed with low nutrition and $10^8$ CFU/mL of Hi188 grew and developed significantly, with a longer body length, a wider eye diameter, and a larger body area, indicating that the Hi188 alleviates to some extent the adverse effects caused by the low protein (Fig. 10), which suggested *Lactiplantibacillus plantarum* Hi188 had a growth-promoting effect on juvenile zebrafish under low protein nutrition background.

**Example 6 Growth-Promoting Effect of *Lactiplantibacillus plantarum* Hi188 in Mice**

Three-week-old male C57BL/6J mice (Shanghai SLAC Laboratory Animal Co., Ltd.) were used as experimental subjects. The duration for the experiment was one week. Each animal in the normal control group (CON), Hi188 group and WJL group were gavaged with 0.2 mL of sterile saline, *Lactiplantibacillus plantarum* Hi188 and *Lactiplantibacillus plantarum* WJL (described in Martin Schwarzer *et al.,*

(2023) Microbe-mediated intestinal NOD2 stimulation improves linear growth of undernourished infant mice. Science Doi: 10.1126/science. ade9767), respectively. and with the dose of Hi188 or WJL was $1 \times 10^9$ CFU /mouse. There were 10 mice in the normal control group and 10 mice in the Hi188 group (n = 10), and 5 mice in the WJL group (n = 5).

At 3 weeks of age, there is no significant difference in body weight and body length among the three groups of mice (Fig.11), whereas at 4 weeks of age, there is a great significant difference between the Hi188 group and the normal control group or the WJL group (Fig.12). These results indicate that the Hi188 strain can promote the increase in body weight and the linear growth of body length in mice.

At 3 weeks of age, there is no difference in the femur and tibia lengths among the three groups of mice, whereas at 4 weeks of age, there is a great significant difference between the Hi188 group and the normal control group or the WJL group (Fig. 13 and Fig. 14). These results suggest that the Hi188 strain can promote the growth of femur and tibia in mice.

The above results demonstrate the growth-promoting effect of the Hi188 strain in mice.

Although Examples of the present disclosure have been shown and described above, it is to be understood that the above-described Examples are illustrative and not restrictive of the present disclosure, and that variations, modifications, substitutions, and alterations to the above-described Examples s may be made by those skilled in the art without departing from the scope of the disclosure.

**Claims**

1. *Lactiplantibacillus plantarum* Hi188, which is deposited with the Accession number of CGMCC No.30250.

2. A formulation comprising the *Lactiplantibacillus plantarum* Hi188 according to claim 1.

3. The formulation according to claim 2, which is one or more of a food raw material, a probiotic powder, a food additive, a feed additive, a pharmaceutical, a liquid drink, a food or a nutraceutical.

4. The formulation according to claim 2 or claim 3, which is a solid or liquid formulation, wherein the formulation comprises the *Lactiplantibacillus plantarum* Hi188 in an amount of $1.0 \times 10^5$ CFU - $1.0 \times 10^{12}$ CFU/mL or $1.0 \times 10^5$ CFU - $1.0 \times 10^{12}$ CFU/g.

5. The formulation according to claim 2 or claim 3, further comprising an excipient, and said excipient comprises one or more of inulin, fructo-oligosaccharides, skimmed milk powder, desalted whey powder, lactoferrin, casein phosphopeptide or hydrolyzed egg yolk powder.

6. Use of the *Lactiplantibacillus plantarum* Hi188 according to claim 1 for the preparation of a formulation or kit for promoting the growth of a subject.

7. The use according to claim 6, wherein the amount of the *Lactiplantibacillus plantarum* Hi188 is $1.0 \times 10^5$ CFU - $1.0 \times 10^{12}$ CFU/mL or $1.0 \times 10^5$ CFU - $1.0 \times 10^{12}$ CFU/g.

8. The use according to claim 6 or claim 7, wherein promoting growth of the subject comprises one or more of promoting an increase in body length or height of the subject, promoting an increase in body weight of the subject, and promoting development of the subject.

9. The use according to claim 6 or claim 7, wherein the subject is a Drosophila, a zebrafish, or a mammal.

10. The use of claim 9, wherein the mammal is a mouse or a human.

Fig. 1

Fig. 2

Fig. 3

Fig. 4

Fig. 5

**A**

normal nutrition          normal nutrition + Hi188

**B**

Fig. 6

Fig. 7

**A**

normal nutrition          low nutrition          low nutrition + Hi188

**B**

Fig. 8

Fig. 9

**A**

normal nutrition     low nutrition     low nutrition + Hi188

**B**

Fig. 10

**A**

the body weight
at week 3

**B**

the body length
at week 3

Fig. 11

A

the body weight at
week 4

B

the body length
at week 4

C

the increase of the body
weight at week 4

D

the increase of the body
length at week 4

Fig. 12

EP 4 674 949 A1

Fig. 13

Fig. 14

## INTERNATIONAL SEARCH REPORT

| International application No. |
| --- |
| **PCT/CN2024/113374** |

| A. | CLASSIFICATION OF SUBJECT MATTER |
| --- | --- |

C12N1/20(2006.01)i; A61K35/747(2015.01)i

According to International Patent Classification (IPC) or to both national classification and IPC

| B. | FIELDS SEARCHED |
| --- | --- |

Minimum documentation searched (classification system followed by classification symbols)

IPC:C12N,A61K

Documentation searched other than minimum documentation to the extent that such documents are included in the fields searched

Electronic data base consulted during the international search (name of data base and, where practicable, search terms used)

CNABS, CNTXT, EPTXT, JPTXT, TWTXT, USTXT, VEN, WOTXT, cnki, 万方, WANFANG, ncbi, 百度, BAIDU, 百度学术, BAIDU SCHOLAR, 中国专利生物序列检索系统, China Patents Biological Sequence Search System, blast, EBI, STN, Uniprot: 内蒙古蒙牛乳业(集团)股份有限公司, 薛正晟, 毛跃建, 张旭光, 杨静, 张美玲, 杨泳美, 朱晨旭, CGMCC30250, CGMCC 30250, 30250, No.30250, No. 30250, CGMCC No.30250, CGMCC No. 30250, CGMCCNo.30250, CGMCCNo. 30250, 植物乳植杆菌, 植物乳杆菌, Lactiplantibacillus plantarum, Lactiplantibacillus, plantarum, lactobacillus plantarum, lactobacillus, Hi188, Hi 188, Hi-188, 体重, weight, SEQ ID NO:3

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| PX | CN 118240712 A (INNER MONGOLIA MENGNIU DAIRY (GROUP) CO., LTD.) 25 June 2024 (2024-06-25)<br>claims 1-10 | 1-10 |
| A | CN 117159598 A (WECARE PROBIOTICS (SUZHOU) CO., LTD.) 05 December 2023 (2023-12-05)<br>description, paragraphs [0008]-[0009] | 1-10 |
| A | CN 115322932 A (TIANJIN UNIVERSITY OF SCIENCE AND TECHNOLOGY) 11 November 2022 (2022-11-11)<br>entire document | 1-10 |
| A | CN 114350578 A (INSTITUTE OF MICROBIOLOGY, GUANGDONG ACADEMY OF SCIENCES (GUANGDONG DETECTION CENTER OF MICROBIOLOGY) et al.) 15 April 2022 (2022-04-15)<br>entire document | 1-10 |

☑ Further documents are listed in the continuation of Box C.    ☑ See patent family annex.

| | |
| --- | --- |
| \*   Special categories of cited documents:<br>"A"   document defining the general state of the art which is not considered to be of particular relevance<br>"D"   document cited by the applicant in the international application<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
| --- | --- |
| **08 February 2025** | **14 February 2025** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**EP 4 674 949 A1**

**INTERNATIONAL SEARCH REPORT**

| International application No. |
| --- |
| **PCT/CN2024/113374** |

| C. | DOCUMENTS CONSIDERED TO BE RELEVANT | |
| --- | --- | --- |
| Category* | Citation of document, with indication, where appropriate, of the relevant passages | Relevant to claim No. |
| A | CN 114672443 A (SHANDONG BAIWO BIOTECHNOLOGY CO., LTD.) 28 June 2022 (2022-06-28) entire document | 1-10 |
| A | WO 2022128927 A1 (DUPONT NUTRITION BIOSCIENCES APS) 23 June 2022 (2022-06-23) entire document | 1-10 |

Form PCT/ISA/210 (second sheet) (July 2022)

25

**INTERNATIONAL SEARCH REPORT**

International application No.

**PCT/CN2024/113374**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

| International application No. |
| --- |
| **PCT/CN2024/113374** |

| Patent document cited in search report | | | Publication date (day/month/year) | Patent family member(s) | | | Publication date (day/month/year) |
| --- | --- | --- | --- | --- | --- | --- | --- |
| CN | 118240712 | A | 25 June 2024 | None | | | |
| CN | 117159598 | A | 05 December 2023 | None | | | |
| CN | 115322932 | A | 11 November 2022 | None | | | |
| CN | 114350578 | A | 15 April 2022 | None | | | |
| CN | 114672443 | A | 28 June 2022 | None | | | |
| WO | 2022128927 | A1 | 23 June 2022 | EP | 4014755 | A1 | 22 June 2022 |
| | | | | US | 2024108668 | A1 | 04 April 2024 |
| | | | | EP | 4262437 | A1 | 25 October 2023 |
| | | | | KR | 20230118939 | A | 14 August 2023 |
| | | | | CA | 3201727 | A1 | 23 June 2022 |
| | | | | AU | 2021399012 | A1 | 22 June 2023 |
| | | | | AU | 2021399012 | A9 | 31 October 2024 |
| | | | | JP | 2023552860 | A | 19 December 2023 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

• **MARTIN SCHWARZER**. Microbe-mediated intestinal NOD2 stimulation improves linear growth of undernourished infant mice. *Science*, 2023